# EUROPÄISCHE PATENTSCHRIFT

(11) **EP 1 474 185 B1**
(45) Veröffentlichungstag und Bekanntmachung des Hinweises auf die Patenterteilung: **03.05.2006**
(21) Anmeldenummer: 03708087.6
(22) Anmeldetag: 10.02.2003
(51) Int. Cl.: A61L 27/30, A61F 2/30, A61L 27/32, A61F 2/36, A61F 2/38, A61F 2/42

(54) **KÖRPERGELENKERSETZENDES TITANIMPLANTAT MIT EINEM ODER MEHREREN GRUNDKöRPERN**
BODY JOINT REPLACEMENT TITANIUM IMPLANT COMPRISING ONE OR SEVERAL BASE BODIES
IMPLANT EN TITANE SERVANT A REMPLACER UNE ARTICULATION DU CORPS ET COMPORTANT UN OU PLUSIEURS CORPS DE BASE

(30) Priorität: 15.02.2002 DE 10206627
(43) Veröffentlichungstag der Anmeldung: 10.11.2004
(73) Patentinhaber: Dot Gmbh, D-18059 Rostock (DE); Diocom B.V., 6721 JK Bennekom (NL)
(72) Erfinder: NEUMANN, Hans-Georg, 18146 Rostock (DE); ZEGGEL, Peter, 18059 Sildemow (DE); MELSOM, Giles, NL-6721 JK Bennekom (NL); HERMSEN, Egidius, NL-5645 EJ Eindhoven (NL)
(74) Vertreter: Garrels, Sabine
(86) Internationale Anmeldenummer: PCT/EP2003/001275
(87) Internationale Veröffentlichungsnummer: WO 2003/068286

(56) Entgegenhaltungen:
- EP-A- 0 232 791
- EP-A- 0 248 117
- EP-A- 1 228 775
- WO-A-00/72777
- US-A- 5 211 833
- US-A- 5 833 463
- US-A1- 2002 099 449

## Beschreibung

Die Erfindung betrifft ein Titanimplantat, das einen oder mehrere Grundkörper aufweist, die sich im Knochen verankern.

In der Orthopädie werden Titan bzw. Titanlegierungen als Werkstoff für die Herstellung von Implantaten verwendet, weil es zum einen leichter als Stahllegierungon, und zum anderen biokornpatibel ist. Dies erlaubt das Verwachsen des Implantats z.B. mit Knochenzellen.

Bei manchen Implantaten ist das Einwachsen jedoch nur zum Teil erwünscht. Um z.B, eine gute Verankerung eines zement freien Hüftimplantates zu erreichen, ist es erforderlich, auf seinem proximalen Teil das Wachstum der Knochenzellen anzuregen, um so eine gute Verbindung zwischen dem Knochen und dem Implantat zu erreichen. Da, wie das Wolffsche Gesetz lehrt, die Neubildung des Knochens dort am Stärksten ist, wo die optimale physiologische Belastung vorhanden ist, besteht die Gefahr, dass der Knochenneubildungsbereich zum distalen Teil des Implantates wandert. In diesem Bereich wird das Knochenwachstrum am stärksten angeregt und das Implantat wird dort am stärksten mit dem Knochen verbunden. Es besteht dann die Gefahr, dass im proximalen Bereich ein Knochenabbau stattfindet und das Implantat sich in diesem Bereich lockert. Dies kann im ungünstigsten Fall zu einem Bruch des Implantates führen. Um die Gefahr der Lockerung des Implantates zu verhindern, weisen Hüftümplantate zum Teil einen besonders augestalteten Proximalbereich auf, der mit einer aufgerauten Oberfläche und/oder Beschichtung aus Calciumphosphaten versehen ist, auf denen das Knochenwachstum gefördert wird bzw. eine gute Verankerung des Knochens erreicht werden soll. Diese Maßnahmen können jedoch eine Knochenneubildung und damit eine Verankerung des Implantates im distalen Bereich nicht verhindern. Um der Lockerung des Implantates im proximalen. Bereich entgegen-zu, wirken, weisen andere Implantate im distalen Bereich polierte Flächen auf. Eine polierte Oberfläche verzögert zwar die Ausbildung von Knochenzellen auf der Metalloberfläche, sie vermag eine feste Verankerung im Knochen jedoch nicht zu verhindern.

Es ist die Aufgabe der vorliegenden Erfindung, die Oberfläche des Bereiches eines Titanimplantates, bei dem ein Verwachsen mit Körpergewebe bzw. Knochenzellen unerwünscht ist, so auszugestalten, dass eine Neubildung von Körperzellen auf der Metalloberfläche behindert wird.

Die Aufgabe wird erfindungsgemäß dadurch gelöst, dass ein Teil des Titanimplantates eine durch anodische Oxidation Typ II hergestellte Schicht aufweist, die Sauerstoff und Silizium enthält.

Das Verfahren der anodischen Oxidation Typ II ist in der AMS 2488, herausgegeben von der SAE International, Warrendale, USA, 1994, beschrieben. Bei der anodischen Oxidation Typ II wird keine Schicht aus TiO₂ ausgebildet, vielmehr werden Sauerstoff und Silizium in die oberflächennahe Schicht, die als Konversionsschicht bezeichnet wird, eingetragen und bilden einen integralen Bestandteil des Materials. Diese Schicht behindert zum einen das Anwachsen von Körperzellen, zum anderen wird durch sie eine erhöhte Dauerfestigkeit des Titanmaterials erreicht. Für ein Hüftimplantat bedeutet dies, dass es in dem anodisch (Typ II) oxidierten Bereich nicht zu einer festen Verankerung des Implantates mit dem Knochen kommt. Der Belastungsbereich, der für die Ausbildung des Knochens erforderlich ist, bleibt daher auf den proximalen Bereich des Implantates beschränkt. Damit wird eine Lockerung des Implantates vermieden. Sollte es langfristig dennoch zu einem Einwachsen im distalen Bereich kommen, wird durch die durch erhöhte Dauerfestigkeit die Gefahr eines Bruches des Implantates verringert. Die vorstehenden Ausführungen gelten auch für Knieimplantate.

Bei Titanimplantaten mit beweglichen Steckverbindungen wird durch die Beschichtung der Steckverbindung ein Einwachsen mit Bindegewebe behindert. Ein weiterer Vorteil besteht darin, dass die Gleitfähigkeit der Steckverbindung verbessert wird, da die Beschichtung ein Kaltverschweißen der beiden Teile der Steckverbindung verhindert

Vorteilhafte Ausgestaltungen des erfindungsgemäßen lmplantates werden in den Unteransprüchen beschrieben.

Im folgenden werden Beispiele der Erfindung anhand von Abbildungen näher erläutert.

Es zeigen:
Fig. I eine schematische Darstellung eines Hüftimplantates und

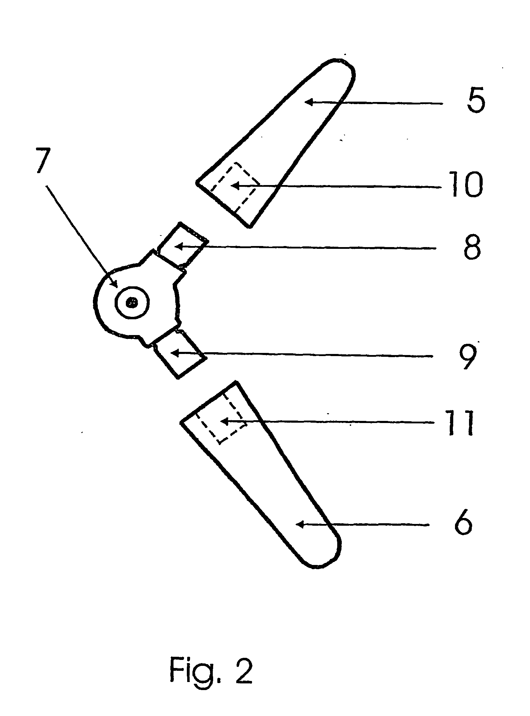

Fig. 2 eine schematische Darstellung eines Fingerimplantates

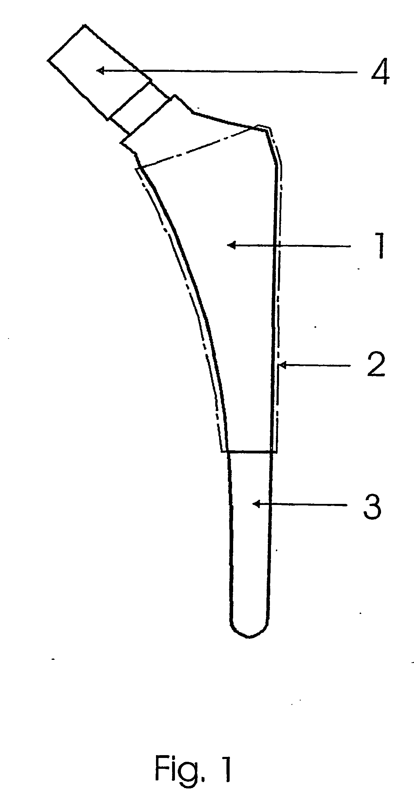

Das Hüftimplantat der Fig. 1 besteht aus einem Grundkörper 1, der in seinem proximalen Teil einen Bereich 2 aufweist, der mit dem Knochen fest verankert werden soll. Dieser Bereich weist bevorzugt eine raue Oberfläche auf, die durch Sandstrahlen mit grobkörnigem Material oder durch eine Titanbeschichtung erzeugt werden kann. Um das Knochenwachstum anzuregen, ist es vorteilhaft, die raue Oberfläche mit einer oder mehreren resorbierbaren Calziumphosphatschichten zu beschichten. Diesem Bereich schließt sich im distalen Teil des Implantates ein Bereich 3 an, der nicht mit dem Knochen verwachsen soll deshalb anodisch
(Typ II) oxidiert wird. Bei dieser Oberflächenmodifizierung werden Sauerstoff und Silizium in die Titanoberfläche eingebracht und bilden dort einen integralen Bestandteil der Konversionsschicht. Der Bereich weist keine TiO₂-Schicht auf. Falls erwünscht, können auch Kohlenstoff und Stickstoff in die Konversionsschicht eingebracht werden.

Die Konversionsschicht behindert zum einen das Anwachsen von Knochenzellen. Sie führt aber auch zu einer größeren Härte und damit zu einer höheren Dauerfestigkeit dieses Teiles und vermindert die Reibkorrosion.

Das Hüftimplantat weist ferner einen Konus 4 für einen Steckkopf auf. Da es bei Revisionsoperationen zum Teil erwünscht ist, dass der Grundkörper des Hüftimplantates im Knochen verbleibt, ist es vorteilhaft, auch den Konus 4 anodisch Typ II zu oxidieren. Es ist dann einfacher, den Steckkopf vom Grundkörper zu entfernen. Zudem schützt die größere Härte vor Beschädigungen des Konus.

In Fig. 2 ist eine Fingergelenksimplantat dargestellt, das aus den Grundkörpern 5 und 6 besteht, die mit den entsprechenden Fingerknochen verwachsen sollen. Sie weisen eine raue Oberfläche auf und sind vorteilhaft mit einer oder mehreren resorbierbaren Calziumphosphatschichten versehen. Verbunden sind die beiden Grundkörper 5 und 6 über ein Gelenkteil 7 mit den Konen 8 und 9, die in Verankerungshülsen, 10, 11, die sich in den Grundkörpern 5 und 6 befinden, gesteckt werden. Um eine Bewegung der Konen 8 und 9 in den Hülsen 10, 11 zu ermöglichen, ist eine bewegliche Anordnung notwendig. Die Beweglichkeit muss auch nach der Implantation des Fingergelenkimplantats gewährleistet sein und darf nicht durch Verwachsen mit Bindegewebe behindert werden. Die Konen 8 und 9 und auch die Verankerungshülsen 10,11 sind deshalb mit einer anodischen Oxidationsschicht Typ II versehen. Außer der Unterdrückung des Anwachsens von Bindegewebe hat die Beschichtung den Vorteil, dass ein Kaltverschweißen der Konen mit den Verankerungshülsen verhindert wird und gute Gleitflächen ausgebildet werden.

## Patentansprüche

1. Ein Körpergelenk ersetzendes Titanimplantat mit einem oder mehreren Grundkörpern (1, 5, 6), die sich im Knochen verankern, **dadurch gekennzeichnet, dass** ein Teil des Implantates (3, 8, 9) eine anodisch (Typ II) oxidierte Schicht aufweist, die Sauerstoff und Silizium enthält

2. Titanimplantat nach Anspruch 1, **dadurch gekennzeichnet, dass** die Schicht zusätzlich Kohlenstoff und/oder Stickstoff enthält.

3. Titanimplantat nach Anspruch 1 oder 2, **dadurch gekennzeichnet, dass** der nicht beschichtete, sich mit dem Knochen verbindende Teil (2, 5, 6) eine raue Oberfläche aufweist.

4. Titanimplantat nach Anspruch 3, **dadurch gekennzeichnet, dass** die raue Oberfläche mit resorbierbaren Calziumphosphaten beschichtet ist.

5. Titanimplantat nach Anspruch 4, **dadurch gekennzeichnet, dass** die Calziumphosphatschicht aus mehren Schichten besteht.

6. Titanimplantat nach einem der Ansprüche 1 bis 5 **dadurch gekennzeichnet, dass** es sich um ein Hüftimplantat handelt.

7. Titanimplantat nach Anspruch 7, **dadurch gekennzeichnet, dass** der Konus (4) für einen Steckkopf ebenfalls eine anodisch (Typ II) oxidierte Schicht aufweist.

8. Titanimplantat nach einem der Ansprüche 1 bis 5, **dadurch gekennzeichnet, dass** es sich bei dem Implantat um ein Knieimplantat handelt.

9. Implantat nach Anspruch 1 bis 5, **dadurch gekennzeichnet, dass** es sich bei dem Implantat um ein Fingergelenk handelt das aus zwei Grundkörpern besteht, die über ein Gelenk miteinander verbunden sind, wobei das Gelenk eine anodisch (Typ II) oxidierte Schicht aufweist.

10. Implantat nach Anspruch 9, **dadurch gekennzeichnet, dass** das Fingerimplantat aus den Grundkörpern (5, 6)und einen davon getrennten Gelenkteil (7) besteht, dessen Konen (8, 9) des Gelenkteils (7) und die Veranlterungshülsen in den Grundkörpern (5, 6) anodisch (Typ II) oxidierte Schichten aufweisen.

## Claims

1. A titanium implant replacing a body joint comprising one or multiple base bodies (1, 5, 6) which are anchoring in the bone, **characterized in that** a part of said implant (3, 8, 9) has an anodically (type II) oxidized layer which includes oxygen and silicon.

2. A titanium implant according to claim 1, **characterized in that** said layer additionally includes carbon and/or nitrogen.

3. A titanium implant according to claim 1 or claim 2, **characterized in that** the uncoated part (2, 5, 6) combining with said bone has a rough surface.

4. A titanium implant according to claim 3, **characterized in that** said rough surface is coated with absorbable calcium phosphates.

5. A titanium implant according to claim 4, **characterized in that** said calcium phosphate layer consists of multiple layers.

6. A titanium implant according to any one of claims 1 to 5, **characterized in that** said implant is concerned with a hip joint implant.

7. A titanium implant according to claim 7, **characterized in that** the cone part (4) for a plug-in head has an anodically (type II) oxidized layer as well.

8. A titanium implant according to any one of claims 1 to 5, **characterized in that** said implant is concerned with a knee implant.

9. A titanium implant according to claims 1 to 5, **characterized in that** said implant is concerned with a finger joint which consists of two base bodies which are connected to each other by means of a joint, wherein said joint has an anodically (type II) oxidized layer.

10. A titanium implant according to claim 9, **characterized in that** said finger implant consists of said base bodies (5, 6) and a joint part (7) separated from it, wherein said cone parts (8, 9) of said joint part (7) and the anchoring sleeves in said base bodies (5, 6) have anodically (type II) oxidized layers.

## Revendications

1. Implant titane remplacant une articulation avec un ou plusieurs élements de base (1, 5, 6) se fixant dans l'os, **caractérisé en ce qu'**une partie de l'implant (3, 8, 9) présente une couche oxidée anodiquement (type II) contenant de l'oxygène et du silicium.

2. Implant titane d'après la revendication 1, **caractérisé en ce que** la couche contient additionellement de carbone et/ou de l'azote.

3. Implant titane d'après la revendication 1 ou 2, **caractérisé en ce que** la partie non-revêtue (2, 5, 6) s'unifiant avec l'os présente une surface raboteuse.

4. Implant titane d'après la revendication 3, **caractérisé en ce que** la surface raboteuse est revêtue par des phosphates de calcium resorbable.

5. Implant titane d'après la revendication 4, **caractérisé en ce que** la couche des phosphates de calcium consiste en plusieures couches.

6. Implant titane d'après les revendications 1-5, **caractérisé en ce qu'**il s'agit d'un implant de hanche.

7. Implant titane d'après la revendication 7, **caractérisé en ce que** le cône (4) pour une tête d'attachement présente également une couche oxidée anodiquement (type II).

8. Implant titane d'après les revendications 1-5, **caractérisé en ce qu'**il s'agit d'un implant de genoux.

9. Implant d'après les revendications 1-5, **caractérisé en ce qu'**il s'agit d'une articulation du doigt consistant en deux éléments de base raccordé par une articulation, tandis que l'articulation présente une couche oxidée anodiquement (type 11).

10. Implant d'après la revendication 9, **caractérisé en ce que** l'implant du doigt consiste en éléments de base (5, 6) et une partie articulaire (7) séparée, tandis que les cônes (8, 9) de la partie articulaire (7) et les manchons de fixation dans les éléments de base (5; 6) présentent des couches oxidées anodiquement (type II).
